# EUROPEAN PATENT SPECIFICATION

(11) **EP 2 508 127 B1**
(45) Date of publication and mention of the grant of the patent: **18.01.2017**
(21) Application number: 11161382.4
(22) Date of filing: 06.04.2011
(51) Int. Cl.: A61B 5/11, G06K 9/00

(54) **Method and system for posture evaluation**
Verfahren und System zur Haltungsbeurteilung
Procédé et système d'évaluation de la posture

(43) Date of publication of application: 10.10.2012
(73) Proprietor: Nederlandse Organisatie voor toegepast- natuurwetenschappelijk onderzoek TNO, 2595 DA 's-Gravenhage (NL)
(72) Inventor: van Rhijn, Gu Johanna Willemina, 2628 VK Delft (NL); Bosch, Tim, 2628 VK Delft (NL); Könemann, Reinier, 2628 VK Delft (NL)
(74) Representative: V.O.

(56) References cited:
- DE-A1- 10 124 242
- US-A- 6 059 576
- US-A1- 2007 169 364
- JAYARAM U ET AL: "Introducing quantitative analysis methods into virtual environments for real-time and continuous ergonomic evaluations", COMPUTERS IN INDUSTRY, ELSEVIER SCIENCE PUBLISHERS. AMSTERDAM, NL, vol. 57, no. 3, 1 April 2006 (2006-04-01), pages 283-296, XP025031567, ISSN: 0166-3615, DOI: 10.1016/J.COMPIND.2005.12.005 [retrieved on 2006-04-01]

## Description

The present invention relates to the field of posture evaluation and feedback, in particular an apparatus and method for generating feedback on a test subject's body posture.

Achieving and maintaining proper body posture, especially during recurring workload conditions, is important for reducing physical stress and avoiding possible injuries on the work floor. Problems resulting from bad body posture occur in almost all branches of the industry, from construction to ICT or office work. In particular, people working on an assembly line are exposed to various stresses on their body, in particular certain body extremities such as their arms and/or neck. Such a worker may be maintaining a static position, holding his arms out at an uncomfortable and possibly harmful angle with respect to his trunk, e.g. while inserting a screw. Also, the worker may be performing repetitive movements whereby he moves his arms repeatedly up and down, e.g. for picking up and attaching a plurality of components. Alternatively or simultaneously an assembly worker may hold his neck in an uncomfortable and possibly harmful static posture, e.g. while performing precision tasks like soldering. Such health risks may occur in many professions, e.g. also dentists or surgeons may experience harmful static neck and/or arm postures, e.g. while performing dental treatment or surgery, respectively.

Methods and systems for global evaluation of human body postures are known in the art. An example is the Ovako working-posture analysis system (OWAS). This system is used to categorize the health risks associated with the position of different parts of the body, e.g. trunk, arms, lower body, and neck. Because the manual monitoring of test subjects can be a time consuming and possibly subjective task, it is advantageous to automate this task. Such automatic systems for monitoring body posture are known in the art, but usually focus on a single aspect or dynamic range of a test subject's body posture.

For example, US 2008/0100459 A1 describes a device worn on the body of a user for maintaining proper posture of the back area. The device comprises a microprocessor that measures a tilt angle from a tilt sensor over a predetermined period and determines an average and a frequency of the angle change. The output control signal is generated only when the tilt angle exceeds a predetermined threshold over the predetermined time period. This known device is suitable for the prevention of a specific type of activity, namely standing too long in a bent position. However, a subject may experience stresses on his body also from other movements that do not fall under these specific thresholds. It is desirable to provide feedback also for other types of activities without having to perform additional measurements.

A problem of the prior art is how to provide automated evaluation for a range of different activities with a minimal number of measurement variables.

### SUMMARY OF THE INVENTION

In a first aspect there is provided an apparatus for generating feedback on a test subject's body posture. The apparatus comprises a memory, a clock, a motion recording device, a processor, and a feedback device.

The memory is arranged for storage and retrieval of motion data and a plurality of parameters used in processing and evaluation of the motion data. The clock is arranged for providing an indication of time. The motion recording device is arranged for recording motion data including an angle between a subject's body extremity and a reference direction to the memory as a function of a time provided by the clock. The feedback device is arranged for generating one or more of a visual, audio, or haptic feedback signal and controlled by the processor.

In operation the processor retrieves the motion data and parameters from the memory. The data is assigned into a first or second subset. The first subset is defined as data wherein the angle remains within a threshold angle bandwidth for a period longer than a first threshold time period. The second subset is defined as data not belonging to the first subset. For data belonging to the first subset and recorded within a first time windows, a fraction of time is calculated wherein the angle exceeds a first angle threshold. For data belonging to the second subset and recorded within a second time window, a number of occurrences is calculated wherein the angle crosses a second angle threshold. The processor is arranged for controlling the feedback device to generate the feedback signal if the fraction of time exceeds a threshold time fraction or the number of occurrences exceeds an occurrence threshold.

In a second aspect there is provided a method for generating feedback on a test subject's body posture. In a step, data is recorded including a time-dependent angle between a test subject's body extremity and a reference direction.

In another step the data is assigned into a first or second subset. The first subset is defined as data wherein the angle remains within a threshold angle bandwidth for a period longer than a first threshold time period. The second subset is defined as data not belonging to the first subset. In another step for data belonging to the first subset recorded in a first time window, a fraction of time is calculated wherein the angle exceeds a first angle threshold; for data belonging to the second subset recorded in a second time window, a number of occurrences is calculated wherein the angle crosses a second angle threshold. In another step one or more of a visual, audio, or haptic feedback signal is generated if the fraction of time exceeds a threshold time fraction or the number of occurrences exceeds an occurrence threshold.

The inventors found that, using the currently disclosed system and method, a single measurement variable, namely the angle between a body extremity and the trunk, can be used to *determine what type* of activity is being performed (e.g. static or dynamic activities) and also be used to *evaluate* that activity using specific thresholds and/or processing that are suitable for that specific activity. It has thus become possible that for feedback on static postures, different criteria be used than for dynamic activities. Furthermore, the inventors have found it advantageous that, for static activities, a threshold for the time duration of the posture be used to provide feedback while, for dynamic activities, a threshold for a frequency of the movement be used.

### BRIEF DESCRIPTION OF THE DRAWINGS

These and other features, aspects, and advantages of the apparatus, systems and methods of the present invention will become better understood from the following description, appended claims, and accompanying drawing wherein:
FIG 1 shows a schematic flowchart of a method for providing feedback on a test subject's body posture.
FIG 2A shows a schematic representation of a test subject with an apparatus for providing feedback on the test subject's body posture, in particular between a test subject's arm and trunk.
FIG 2B shows a schematic representation of a test subject with an apparatus for providing feedback on the test subject's body posture, in particular between a test subject's head/neck and trunk.
FIG 2C shows a visualization of a test subject's body posture recorded by a 3D motion tracking device.
FIG 3 shows a graph of raw data comprising a time-dependent angle between a test subject's body extremity and trunk.
FIG 4 shows a graph of filtered data comprising a time-dependent angle between a test subject's body extremity and trunk.
FIG 5 shows a graph of peaks in the data illustrating possible trigger criteria for a feedback signal.
FIG 6 shows a schematic flowchart of an apparatus for providing feedback on a test subject's body posture.
FIG 7 shows distributions of an angle between a test subject's body extremity and trunk in two subsets of the data.

### DETAILED DESCRIPTION

The following detailed description of certain exemplary embodiments is merely exemplary in nature and is in no way intended to limit the invention, its application, or uses. The description is therefore not to be taken in a limiting sense, and the scope of the present system is defined only by the appended claims. In the description, reference is made to the accompanying drawings which form a part hereof, and in which are shown by way of illustration specific embodiments in which the described devices and methods may be practiced. These embodiments are described in sufficient detail to enable those skilled in the art to practice the presently disclosed systems and methods, and it is to be understood that other embodiments may be utilized and that structural and logical changes may be made. Moreover, for the purpose of clarity, detailed descriptions of well-known devices and methods are omitted so as not to obscure the description of the present system.

Systems for measuring body posture are known in the art, e.g. NL1011845, WO2007/015134, US2008/100459, WO2009/112281 and US2007/0169364.

These known systems do not provide a means for automatically assigning measured data into static and dynamic subsets, e.g. based on peak detections of the angular data, nor do these known systems provide a feedback signal based on separate evaluation ranges and thresholds for these different types of activity, based on this same angular data. Further advantages and areas of applicability of the present systems and methods will become apparent from the detailed description provided hereinafter.

The currently proposed measurement and processing methods can be used for automatically assigning measured data of a subject's body posture and movement into static and dynamic periods based on a single (time-dependent) measurement variable. Depending on this assignment, different evaluation ranges or data processing can be used on this same data in the different periods ranges or data processing can be used on this same data in the different periods to assess whether a body posture or movement has a harmful effect on the subject's condition. It is found that harmful effects for the subject may occur for both static posture and dynamic movements, but that the evaluation range and/or data processing can be different.

The term 'posture' is to be interpreted as the overall position and (relative) orientation of a subject's body extremities such as the limbs, and head. In the current method an aspect of a subject's posture that is measured may be an angle between the subject's (upper) arm and a reference direction e.g. along a length of a trunk or a gravitational (downward) vector, e.g. using a tilt sensor. Alternatively or in conjunction, an angle may be measured between the subject's head and a reference direction e.g. along a length of the trunk or a gravitational vector. In particular these angles can be used e.g. to estimate an amount of strain on the subject's shoulder and neck area. Other angles that may be measured to evaluate the posture include the angle of the elbow, wrist, or knee. In general the body extremity may be one or more of a head, arm, lower arm, hand, finger, leg, lower leg, or foot.

A definition of the arm angle could be the angle between a vertical axis (gravitational vector) through the shoulder joint and the upper arm or neck. This angle for the arm is e.g. 0 degrees when a subject is holding his arms alongside his body, 90 degrees when he is holding his arms straight forward, sideward or backward and 180 degrees when he is holding his arms straight up. Similarly, the angle for the head or neck is e.g. 0 degrees when a subject holding his head straight up (in line with his trunk) and a higher angle for movements of the head forward, backward and/or side to side. It is noted that a neck may not move as a single straight rod in any direction, rather it may curve towards a direction. Nevertheless an angle may be defined e.g. by using three markers placed on positions of the head or upper neck, lower neck, and lower on the trunk below the neck. A more general definition of the angle is a relative angle between a direction along a length of the body extremity and along a length of a connecting body part or gravitational vector.

In this text, mention will be made of the measurement of an angle between a subject's arm and body. This angle can be measured, on one arm only, on both arms separately, or on both arms together, e.g. a feedback can be generated if either of the arms exceeds a given threshold, or it can be generated if on average a threshold is exceeded. Where in this text there is a mention of exceeding or crossing a certain threshold angle it is to be understood that this may depend on the definition of the angle. A suitable definition may be one wherein an angle of zero degrees is assigned when the body extremity experiences minimal stress, i.e. a position of rest and/or a natural equilibrium position. Thus when it is mentioned that a certain threshold angle is exceeded this means that a certain threshold for stress on that body extremity is exceeded, e.g. an arm lifted too high, a neck bent too much etc.

For example, a static posture wherein a subject holds his arms up with an angle of more than 60 degrees (plane angle) for more than 4 seconds may be considered harmful (or discomforting) while a dynamic movement wherein the same test subject moves his arms up and down within 4 seconds may be considered harmful when this angle exceeds 60 degrees and exceeds a movement frequency of more than twice per minute. It is thus recognized that static and dynamic postures can have different criteria for evaluating their harmfulness. The current system and method provides a straightforward way of determining whether a posture is static or dynamic and can provide automatic feedback suitable for either instance, e.g. providing a warning signal to the user.

The thresholds for evaluating body posture may result e.g. from clinical studies that are usually performed manually and/or are generalized measurements independent of the specific task for which a control subject is to be evaluated. E.g. it is found that an advantageous first threshold time period for classifying a posture as 'static' is longer than 4 seconds, i.e. a posture wherein a subject is holding his arms within a first threshold angle bandwidth for at least 4 seconds can be classified as static. Furthermore an advantageous second threshold time period for classifying a movement as 'dynamic' is between 0 and 4 seconds, i.e. posture wherein a subject is moving his arms within a period of 0 to 4 seconds over more than a second threshold angle or angle bandwidth. Alternatively any movement may not classified as static may be classified dynamic or vice versa.

In an advantageous embodiment, there is provided a method for obtaining the thresholds for a specific task. In this embodiment, the first and second evaluation ranges are determined by a procedure wherein first data is recorded on a plurality of control subjects performing a similar activity as the activity for which the test subject is to be evaluated. From this recorded data, first and second distributions respectively of the angles in the first and second subsets of the data are determined. Finally, the first and second evaluation ranges are set such that these ranges cut the respective first and second distributions in predetermined relative portions.

In a further advantageous embodiment, the first and second evaluation ranges are determined from a correlation between a control subject's statistical chance for body injury and the control subject's first and second distributions. E.g. if it is determined that statistically more injuries occur for workers moving dynamically at angles greater than 60 degrees (but not e.g. 40 degrees) for on average more than 2 times per minute, or for angles greater than 20 degrees for on average more than 10 times per minute. The evaluation ranges wherein a feedback is generated could be set accordingly to e.g. 20 and 60 degrees and higher, depending on the frequency of these movements. It is noted that an evaluation 'range' need not be limited on both sides, e.g. the range could comprise only a lower or upper limit.

The currently proposed system is thus suitable for evaluation of tasks on a group level, e.g. by automatically creating distributions of static/dynamic movements for different members in a work group and setting the evaluation ranges in accordance with these measurements. E.g. by observing that 90 percent of the control subjects assume only angles lower than a range R1 or R2, such an evaluation range can be set to provide feedback to the other 10 percent of the group members.

The currently proposed automated posture evaluation system and method registers and may evaluates during a certain time window the physical working postures and movements of a subject performing a specific task. This time window may range from just few minutes or less for a direct or real-time feedback to a whole shift or working day for feedback afterwards. Also combinations are possible wherein the worker receives both direct feedback and an overall evaluation. The tool can provide feedback on the level of strain for the test subject. From this feedback, possible long term health risks can be predicted and/or avoided. The current system is particularly suited for workload conditions that involve low forces and high-frequency repetitive movements.

In particular the current system can automatically register and process the angles of the body in time (e.g. amplitude, frequency, and time period). It can discern fast angle changes ('dynamic') and periods wherein the angle barely changes ('static'). This can be done e.g. using peak detection and time measurements. The difference between static and dynamic and the information on the angles that were recorded can be used to assign the data into different subsets and evaluate the data according to the subset.

The currently proposed method provides a full evaluation of the performed task, i.e. both static and dynamic periods are evaluated. A straightforward and easily implemented method is provided for splitting a time-series of measurements into static and dynamic components. The method is objective (not dependent on an observer), can be performed real-time, without interference of an observer (which may influence the task by his presence), and can be performed remotely. The currently proposed method automates a complex evaluation of postures and movements that traditionally involve manual labor by an observer. The method provides a cheaper, more reliable, and faster way for evaluating physical workloads. This knowledge may be of importance e.g. to advisors, insurance companies, etc. that can use the method to screen test subjects and build up knowledge, suggest improvements, reduce risks, increase sustainable performance etc. The system and method may thus prove beneficial in such fields as the manufacturing industry, insurance branch, health inspection, logistics, advice bureaus etc.

Using the currently proposed methods and system it is thus possible to deduce static postures and dynamic movements as well as the corresponding angles. Processing steps may e.g. include one or more of
- a filtering step wherein only peaks/troughs are kept/evaluated; it is noted that the word "peak" used in this text may refer to both high and low extreme values in the data, i.e. minima and maxima in the data;
- an assignment step wherein periods are assigned to subsets according to whether the angle stays 'the same' (within tolerance) to discern static/dynamic periods; For example a static period can be defined as one wherein the angle stays the same for at least 4 seconds while a dynamic period is a rapid back and forth movement (peak surrounded by troughs);
- a further filtering step wherein the movement patterns are further reduced as a function of the assignment (e.g. filtering out movements that do not cross a threshold bandwidth)
- an analysis/calculation step to determine if the data assigned to the subsets falls within preset evaluation ranges for those subsets.

Further advantages and applications may become more apparent from the following detailed description of the drawings. This description again is to be regarded in an illustrative and non-limiting manner. In particular, steps and/or parts of the shown embodiments may be omitted and/or added without departing from the scope of the current methods and systems, which scope is defined by the appended claims.

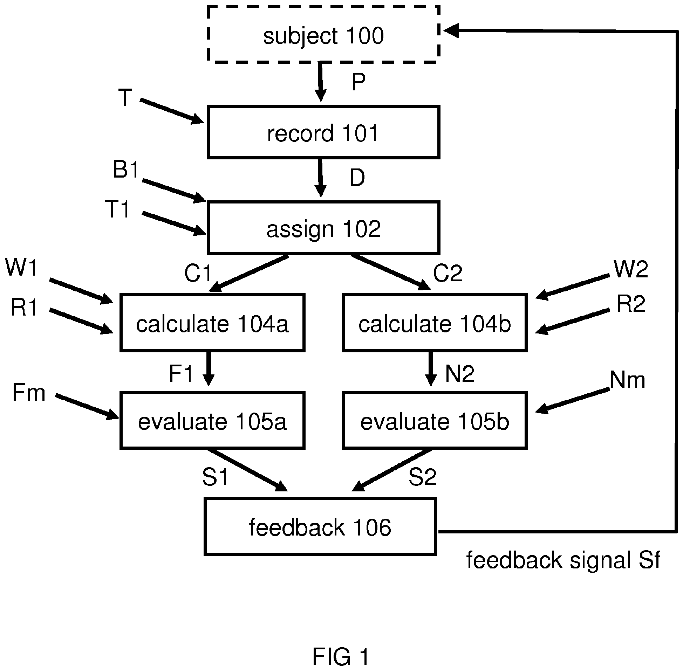

FIG 1 shows a schematic flowchart of a method for providing feedback on a test subject's body posture. From top to bottom, a test subject or user 100 performing an activity will assume different body postures P during a measuring time T. In particular the current method records 101 an angle between the test subject's (upper) arm and body as a function of time. This angle can be e.g. a forward angle of a person holding his arms forwards, or a sideways angle of a person holding his arms sideways.

The recording 101 produces data D that comprises a time-dependent angle between a test subject's body extremity and a reference direction. The data is subsequently assigned 102 into subsets of data C1 or C2, pertaining to static or dynamic periods in the test subject's body posture, respectively. The subset C1 of a static period is defined as data wherein the angle remains within a threshold angle bandwidth B1 for a period longer than a first threshold time period T1. The subset C2 of a dynamic period can be defined as data not belonging to the first subset C1. That way all data will belong to either the static or dynamic subset.

Alternatively, the second subset may be defined e.g. as data wherein the angle is increased and/or decreased repeatedly (i.e. more than one consecutive movement) over more than a second threshold angle bandwidth B2, whereby each increase and/or decrease is performed within a threshold time period that is shorter than the first threshold time period T1. Advantageously it was found that this simple analysis of a single (time-dependent) variable may suffice to assign the test subject's movement pattern into static or dynamic periods.

For example a static period can be defined as a period wherein a subject hold his arms within a 10 degrees angle bandwidth (e.g. between 80 and 90 degrees) for more than 4 seconds (first threshold time period). In the same example, a dynamic period can be defined as a period wherein the subject moves his arms up and down repeatedly over more than 10 degrees. A similar definition for static/dynamic periods can be made for the movement of the neck, although the angle variation and thresholds will be different.

From the categorized data C1, representing a static period, a fraction of time F1 may be calculated in step 104a wherein the angle exceeds an angle threshold R1 (or is below the threshold or in a certain threshold range depending on the definition of the angle) over a first time window W1. The first time window W1 may be a moving window, e.g. the last 60 seconds. When a threshold angle R1 is exceeded say for 30 seconds within the time window of the last 60 seconds, the fraction F1 would be 30/60=0.5. If this fraction F1 is evaluated in step 105a to be above a certain threshold time fraction Fm, e.g. 0.4 for the above example, a control signal S1 may be provided that triggers a feedback 106 in the form of a feedback signal Sf to the user. In stead of a fraction F1 and threshold fraction Fm of course also an absolute time and threshold time may be used in combination with a certain time window. E.g. a feedback may be generated if the threshold angle R1 is exceeded for more than 30 seconds within the last 60 seconds.

From the categorized data C2, representing a dynamic period, a number of occurrences N2 may be calculated in step 104b wherein the angle crosses a certain threshold angle or range R2 within a second time window W2. The second time window W2 may be different from the first time window W1 or may be the same. E.g. the second time window is 5 minutes. A number of occurrences that crosses a threshold angle R2 may be calculated that were recorded in the last 5 minutes. If this number N2 is evaluated in step 105b to exceed an occurrence threshold Nm, a control signal S2 may be provided that triggers a feedback signal Sf to the user. It is thus illustrated that the user may receive feedback 106 for both his dynamic and static periods.

The feedback signal Sf may comprise any form of sensory feedback e.g. one or more of a visual, audio, or haptic (tactile) feedback signal. The feedback signal could be provided during the activity or afterwards. E.g. a feedback signal can be generated while the activity is performed (real-time) and at the moment it is determined that the specific conditions for feedback are fulfilled. E.g. a vibration device or audio signal may be triggered by the specified condition that a subject is holding his arms too high for a period longer than 4 seconds. Alternatively, the feedback can be provided after a period of data accumulation and/or after the subject has finished an activity. E.g. the display of a graph of the subject's activity wherein the fractions are compared to the respective fraction thresholds may be regarded as a form of feedback.

In an alternative embodiment, the categorized data C1 and C2 can be each quantified, e.g. by assigning the data points to a histogram or distribution. For example, a histogram can be divided in bins between 0 and 180 degrees (e.g. 18 bins of size 10). Each data point belonging to the first or second subsets of the data can be used to fill the bins based on the angle of that data point. The assignment of the data point results in a histogram that quantifies the distribution of angles in either the first or second subset of the data. The distribution may represent a relative occurrence of an angle within the respective subset of the data. The histogram or distribution may be normalized e.g. to the total number of data points or the number of data points in the respective subset.

In a subsequent calculation step 104 a first and second evaluation range R1 and R2 can be used to calculate respective fractions of the data points in the first and second subsets C1 and C2 of the data that fall within the respective evaluation ranges R1 and R2. It is thus noted that in this more general example, an evaluation range is a more general alternative for a threshold angle, e.g. instead of using a single angle and only evaluating the data if it is above the threshold, the evaluation range can serve to also evaluate data points falling within a range which may have upper and lower bounds. E.g. a first evaluation range may be chosen to be above 20 degrees. A first fraction is then the fraction of data points that qualify for said evaluation range, i.e. wherein the test subject hold his arms statically (e.g. as defined above) higher than 20 degrees (angle e.g. as measured with respect to a vertical axis through the shoulder jointor as a relative angle between a length of the arm and a length of the trunk).

In a subsequent evaluation step 105 the fractions can be compared to specified fraction thresholds, to evaluate whether or not a feedback signal Sf is to be generated in a subsequent feedback step. E.g. a feedback can be generated if the first fraction exceeds 10 percent or the second fraction exceeds 15 percent. This would mean e.g. that if 10 percent or more of the data points of the first subset fall within the first evaluation range a feedback signal is generated. This feedback signal would also be generated if 15 percent or more of the data points of the second subset falls within the second evaluation range. The method could thus be adjusted e.g. if a test subject hold his arms statically for more than 10 percent of the time at an angle higher than 60 degrees or if the subject moves his arms more than 15 percent of the time higher than 90 degrees. It is noted that when the fraction threshold is set low enough, any angle falling within the evaluation range may trigger a feedback signal. In that sense the fraction threshold forms a more general alternative to the instantaneous feedback on any angle exceeding a threshold value. This use of fractions may be advantageous in particular for evaluating static period.

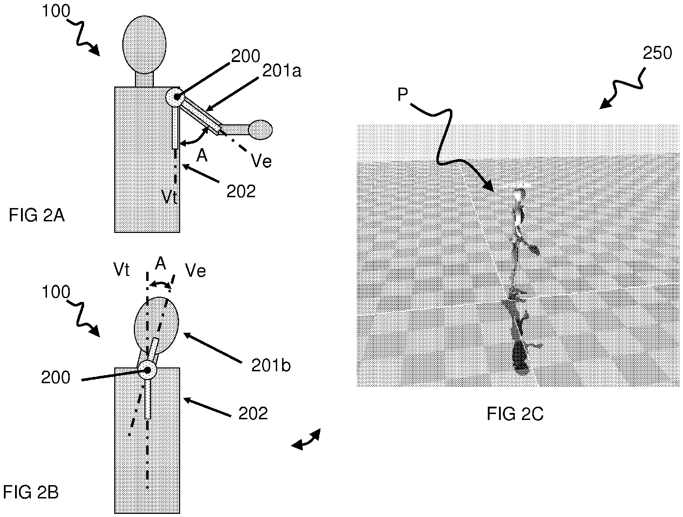

FIG 2A shows a schematic representation of a test subject 100 with an apparatus 200 for providing feedback on the test subject's body posture. The apparatus 200 comprises a means for measuring and recording an angle A between the test subject's arm 201a and trunk (body) 202. This means can be provided e.g. by an apparatus that provides an electronic signal representative of an opening angle between two arms or levers of the apparatus 200. The levers of the apparatus can be attached to the test subject's arm and body, in particular in a direction Vt along the length of the trunk (in this case downwards) and in a direction Ve along the length of the (upper) arm. Alternatively, the angle measuring apparatus could comprise tilt sensors attached along the subject's arms and body for measuring a relative tilt angle between the arm and body.

FIG 2B shows another schematic representation of a test subject 100 with an apparatus 200 for providing feedback on the test subject's body posture. In this case feedback is provided on another body extremity, namely the head 201b. A similar device 200 may be used to measure the angle difference between a direction along a length of the head or neck Ve and a direction Vt along a length of the trunk. It is noted that as shown in the figures 2A and 2B, the angle A in the case of a head or neck measurement may be defined differently than for the arm measurement. In particular the angle for a neck measurement can be defined to be zero when the test subject hold his head straight up, i.e. in line with his trunk. An angle of the neck may be defined to be the angle of moving the head to the front or back or alternatively side to side. In a more general definition any deviation from the normal (0 degree) angle may be considered as an angle of the neck.

FIG 2C shows a visualization 250 of a test subject's body posture P recorded by a 3D motion tracking device. Such a motion tracking device may comprise e.g. a special suit, worn by the user, which suit tracks the relative and/or absolute orientation of various body parts inside the suit. E.g. US5744953 discloses a magnetic motion tracker with transmitter placed on tracked object. The configuration is intended to be suitable to wear on the human body in an ambulatory manner. The configuration contains magnetic field sensors which detect a magnetic field generated by a transmitter positioned on the body.

EP1959831 discloses a motion tracking system for tracking an object composed of object parts in a three-dimensional space, the system comprising magnetic field transmitters and receivers as well as inertial measurement units for recording a linear acceleration, and angular velocity transducers for recording angular velocities. The system further comprises a processor for controlling the transmitters and receiving signals coming from the field receivers and the inertial measurement unit; which processor contains a module for deriving orientation and/or position information of the constituent object parts of the object on the basis of the received signals.

US2008285805 discloses a system for capturing motion of a moving object via a plurality of motion sensor modules placed on various body segments. The sensor modules capture both 3D position and 3D orientation data relating to their respective body segments, thereby gathering motion data having six degrees of freedom with respect to a coordinate system not fixed to the body. Each body sensor collects 3D inertial sensor data and, optionally, magnetic field data. In embodiments, either DSP circuitry within the sensor modules or an external computing device, processes the sensor data to arrive at orientation and position estimates by using an estimation algorithm, such as a Kalman filter or a particle filter. The processing includes biomechanical model constraints that allow flexibility in the joints and provide characteristics for various joint types. To improve estimation accuracy, the system may be integrated with various types of aiding sensors.

Alternatively, the method of motion capture may comprise active or passive markers attached to the body for tracking the motion of specific points of the body using one or more external cameras recording the markers. In the current application a marker may be attached e.g. to the body, shoulder, head and upper arm to measure the desired angle between arm and trunk or neck and trunk. Vicon, Optotrack and Optitrack are examples of commercially available camera tracking systems.

Using other devices for motion capture it is also possible to record a test subject's posture without the use of any markers on the body. Such a device may comprise e.g. two or more camera's that capture different perspectives of a test subject and convert these perspective images in a three dimensional projection of the test subject's posture. Image recognition software could e.g. automatically determine what parts of the recorded image are the arm and body of the test subject. From the recorded posture it is then likewise possible to extract the relevant body angles. Advantageously an external motion tracking device provides a means for measuring a body posture without contacting the test subject. This has an advantage that the test subject may perform his activity without being disturbed by any appended measurement devices.

Such 3D (three-dimensional) motion capture devices are known in the art. For example US 2004179095 discloses a system for 3D scanning of an input image. The system uses an optical grating and two digital cameras to capture the images and then synthesizes these images into a 3D form using software scanning technology.

In an advantageous embodiment the angular data of the position of the subjects arm with respect to his body is thus provided by a contactless angle measuring device such as a remote sensor (e.g. a camera) that records data on the test subject's body posture without contacting the test subject and extracts from the recorded data (e.g. by image recognition software or conversion logic) on the test subject's body posture, data comprising the time-dependent angle between the test subject's arm and body. In a further advantageous embodiment the contactless measuring device comprises at least two imaging devices spatially separated to capture two or more perspective images of the test subject. In another embodiment the data is provided by a motion capture device arranged for recording the test subject's three-dimensional body posture and extracting from the recorded body posture the angle between respectively the test subject's arm and trunk or and the neck and trunk.

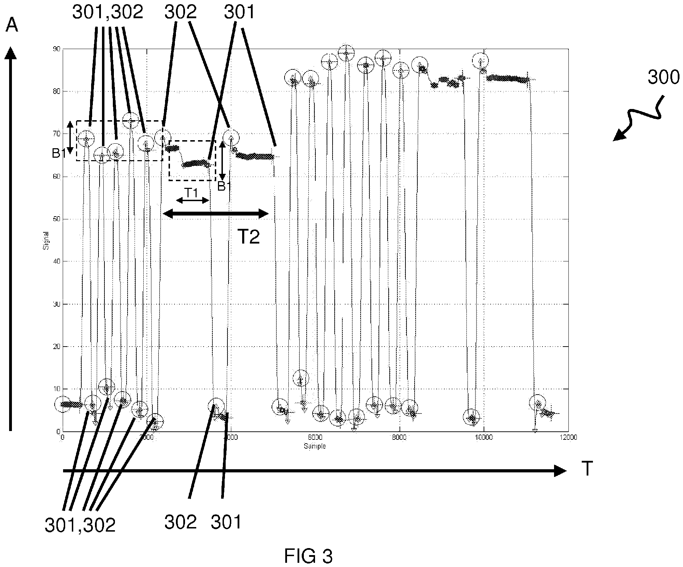

FIG 3 shows a graph 300 of raw data comprising an angle A between a test subject's arm and trunk as a function of time T. In this case the sampling rate of the angle was 120 Hertz, meaning that the 120 angles were recorded per second. The graph represents the activity of a test subject picking up and placing a number of objects from a lower to an elevated position. The data was recorded by an angle measuring device such as discussed above. In the graph 300, indications are given of possible bandwidth and time thresholds B1 and T1, respectively. These thresholds determine if a period will be classified as static or dynamic. Also shown are data points 301, 302 that directly precede or follow a change in angle that exceeds the bandwidth thresholds (also indicated by cross and circle markers around the data points).

In an advantageous embodiment a feedback signal is also generated if the angle A remains within the threshold angle bandwidth B1 for a period longer than a second threshold time period T2. In this case e.g. it could be indicated to the user that he has exceeded the instantaneous time threshold period for a static posture. Note that this time period is longer than the period T1 which may be acceptable for an isolated period, but not if it's occurrence exceeds a threshold fraction of the time. Any combination of the time threshold T2 and/or a threshold average angle Aa may be used. E.g. a lower threshold angle could be used if a test subject exceeds the time period T2, while for the time period T1 used to classify the period as static is used in combination with a higher threshold. In a more embodiment there can be a table or function indicating for each angle a maximum acceptable static time period.

In a further advantageous embodiment a feedback signal is also generated if a frequency of the repeated increase and/or decrease in angle in the second subset (i.e. dynamic movements) exceeds a frequency threshold. In this case a frequency of the movement would be the trigger for the feedback signal, in conjunction with the threshold fraction of the time that the subject is exhibiting dynamic movements in the second evaluation range. The frequency can be defined e.g. as the inverse of a time period between a minimum and maximum peak angle, the peak angle defined as an extreme angle in the angle variation directly following a variation of the angle that is more than a threshold angle variation value. Of course any combinations of the frequency measurement and/or the peak amplitude measurement is possible, e.g. a feedback signal may be generated on the basis of the frequency measurement alone, or a combination could be used wherein a frequency is only measured for peak amplitudes exceeding a predetermined threshold.

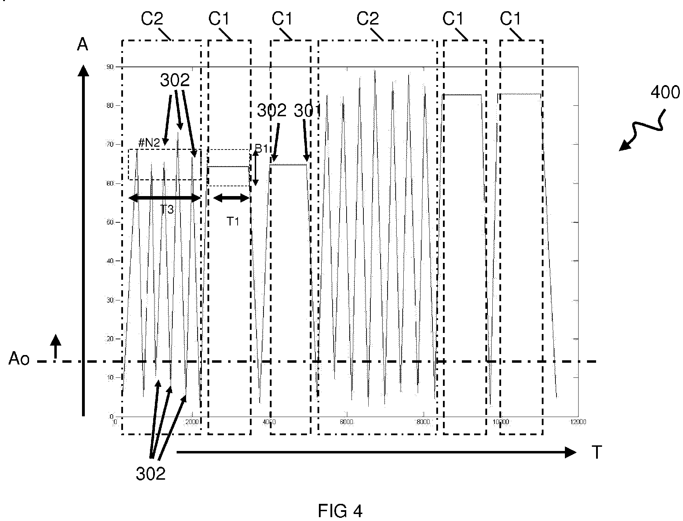

FIG 4 shows a simplified graph 400 of the same data as FIG 3, but filtered to contain only the peaks and plateaus of the raw data set. Such a simplified graph may be obtained e.g. by connecting the points that follow a movement in the dynamic periods C2 and connecting points that follow the last and precede the next movement in the static periods C1. All other data points may be discarded. The static and dynamic period C1 and C2 may be assigned in the data based on the criteria provided above, i.e. a period can be classified as static if the angle stays within a bandwidth B1 for a period longer than T1 and a period can be classified as dynamic if the angle changes repeatedly (e.g. back and forth) at least by #N2 times within a time T3.

In an advantageous embodiment a method is provided wherein the data is filtered prior to the calculating step to store only data points that immediately precede or follow a change in angle that is more than the first angle bandwidth B1 within the first threshold time periods T1.

In a further advantageous embodiment a method is provided wherein a feedback graph is generated on a display, the graph representing a simplified or filtered movement pattern of the test subject obtained by displaying only data points that are used for classifying the data in the first or second subsets C1, C2. An example of such a filtered movement pattern is shown in the graph of FIG 4.

The criterion for static and/or dynamic periods can also be narrowed by adding the prerequisite that the movement needs to comprise an angle greater than a minimum offset angle Ao. This can be used to filter out periods wherein the test subject is not performing any activity, e.g. holding his arms down in a position of rest. In an advantageous embodiment a method is provided wherein the first (static) threshold time period T1 is initiated only when the subject holds his arm higher than a minimum offset angle Ao (e.g. 15 degrees)..

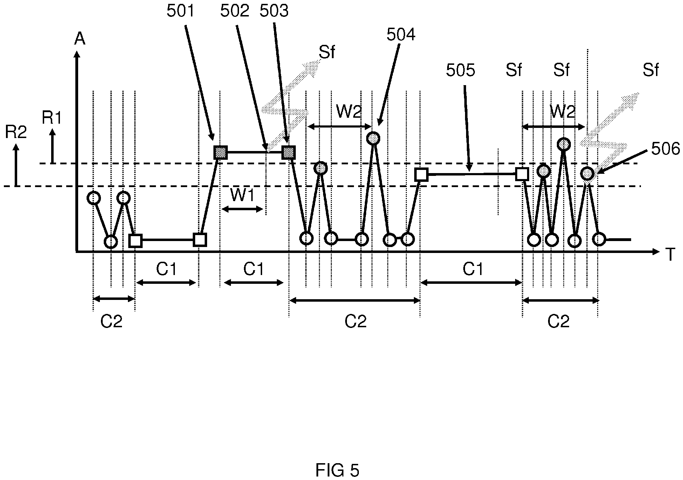

FIG 5 shows a graph of peaks in the data illustrating possible trigger criteria for a feedback signal Sf. The data comprises an angle A that changes as a function of time T. The data is assigned to subset C1 (static) if the angle A stays within a bandwidth B1 for a period longer than threshold time period T1. Data points belonging to subset C1 are indicated with square markers. Data not belonging to subset C1 is assigned to subset C2 (dynamic) and indicated with round markers. Also shown are threshold ranges R1 and R2, i.e. the thresholds angle R1 for the static periods C1 and the threshold angle R2 for the dynamic period C2. The intermediate point 505 may still be used, e.g. for calculating an average angle during a static period. Alternatively these data points may be filtered out as was explained earlier with FIG 4.

Starting with point 501, a static period is determined after a threshold time period is exceeded. When the angle exceeds the respective threshold R1 for more than a preset threshold fraction of the time of a time window W1, a feedback signal Sf is generated (in this case real-time) the moment that this is determined at point 502. In particular, this feedback may be provided before the end of the static period 503. In some instances, this feedback signal may also be delayed, e.g. in case of a dentist or surgeon it may be advantageous to provide feedback after the activity is finished. In that case a time window W1 may be longer or may also be dynamic, e.g. starting and ending with the activity. Point 504 indicates a peak angle that exceeds the respective threshold R2, resulting in a count for the number of dynamic occurrences. Because this number in this case is still below an occurrence threshold (which for this example has been set to 3) no feedback signal is generated..

Finally, during a time window W2, the number of peaks may be measured and a feedback signal Sf generated if this exceeds a specified number, in this case at point 506. The feedback signal is thus generated when a frequency of the dynamic movement exceeds a respective frequency threshold. Of course this could be expanded and combined with the other thresholds, e.g. only movements over more that a second bandwidth threshold are used for determining a maximum frequency, and/or for different movement angles, different frequency thresholds can be used. It is noted that in the current example an occurrence is defined a back and forth crossing of the second angle threshold R2. Alternatively any crossing may be counted and an appropriate occurrence threshold may be set. E.g. in the last time window W2 the angle threshold R2 is crossed 6 times (7 including the last down going angle). To prevent jitter noise wherein an angle is on the edge of a threshold and triggers multiple crossings, a minimum angle bandwidth may be used that must be crossed. E.g. in stead of a single angle threshold R2 and angle range R2 may be used that must be crossed to trigger an occurrence.

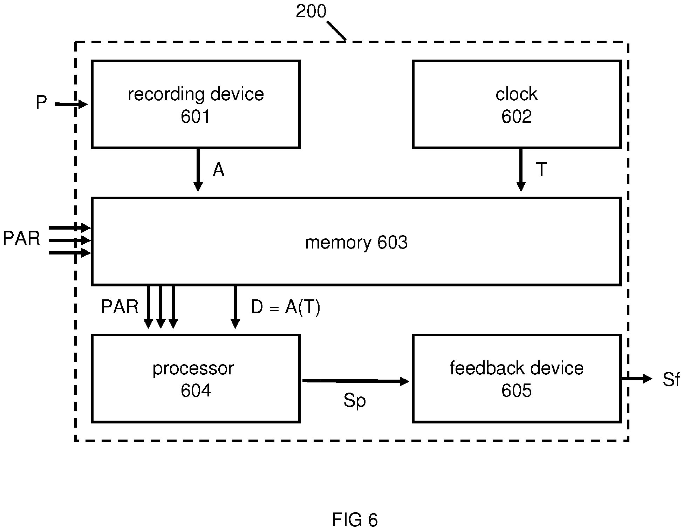

FIG 6 shows a schematic flowchart of an apparatus 200 for providing feedback Sf on a test subject's body posture P. The subject's (body) posture P is recorded by a (motion) recording device 601 which converts it to an angle A that is stored in memory 603 in conjunction with a time stamp T provided by a clock 602. The thus recorded time dependent angle A(T) forms the data D that can be read out by the processor 604 from the memory 603 together with other parameters PAR, which can be provided to the memory separately. These parameters PAR e.g. comprise the various thresholds, bandwidths, and evaluation ranges needed to process the data D. Thus the parameters PAR could comprise e.g. the external parameters shown also in FIG 1. If the processor determines that e.g. an average angle, peak angle, time duration, or movement frequency exceeds a respective threshold for either the static or dynamic periods (which can also be determined by the processor in accordance with the above given criteria for a method of determining static/dynamic postures), a processor control signal Sp is given to the feedback device 605. Upon receiving this signal Sp, the feedback device can provide a feedback signal Sf, e.g. to the user.

The memory 603 may be any suitable type of memory where data are stored, (e.g., RAM, ROM, removable memory, hard drives, floppy disks, memory cards, etc.) or may be a transmission medium or accessible through a network (e.g., a network comprising fiber-optics, the world-wide web, cables, or a wireless channel using time-division multiple access, code-division multiple access, or other radio-frequency channel). Any medium known or developed that can store and/or transmit information suitable for use with a computer system may be used as the computer-readable medium and/or memory. The memory may also store application data as well as other desired data accessible by the controller/processor 604 for configuring it to perform operational acts in accordance with the present systems and methods. Any type of processor may be used such as dedicated or shared one.

The processor 604 may include micro-processors, central processing units (CPUs), digital signal processors (DSPs), ASICs, or any other processor(s) or controller(s) such as digital optical devices, or analog electrical circuits that perform the same functions, and employ electronic techniques and architecture. The processor is typically under software control for example, and has or communicates with a memory that stores the software and other data such as user preferences, parameters, evaluation ranges, and/or time, bandwidth, and fraction thresholds.

Clearly the measurement device 601, clock 602, memory 603, processor 604, and feedback device 605 may all or partly be a portion of a single (fully or partially) integrated unit 200 such as any wearable or contactless device. Alternatively, instead of being integrated in a single device, parts may be distributed between multiple devices, e.g. a separate measurement and/or feedback device.

In an advantageous embodiment the motion recording device comprises a 3D motion tracking device arranged for recording the test subject's three-dimensional body posture and a processor arranged for converting the three-dimensional body posture into a measurement of the angle between the test subject's arm and body. Examples of such devices were provided above. In another embodiment the motion recording device may comprise e.g. a tilt sensor that measures an angle relative to a gravitational vector.

In yet another advantageous embodiment, the test subject is a virtual person and the apparatus 200 further comprises a simulation module (not shown) coupled to the motion recording device 601. For example, the simulation module may be arranged to generate the motion data as input for the recording device and or write this data directly to the memory 603. The simulation module may run a program based on a model of the virtual person performing tasks for which an evaluation is desired. E.g. a simulation may be run wherein the virtual person performs a certain task and the angles of this virtual person's body posture are inputted into the recording device similar as for a real person. The clock may also be coupled to the simulation module so that a simulation may be performed faster than real-time. Further processing may be similar as was explained above, i.e. data may be categorized into static and dynamic periods and separately evaluated.

In another advantageous embodiment the feedback device comprises a display device providing the user with visual feedback signal. The display could e.g. show a feedback signal indicating the harmfulness and/or health risks associated with an evaluation of his activity according to the methods and systems discussed above. In another embodiment, the feedback device 605 may comprise a vibration device and/or a sound-generator arranged for providing the user with a haptic and/or audio feedback signal, respectively. In particular, these types of feedback signals provide and advantage in that they do not require the active attention of the test subject. E.g. the vibration device can be attached to the user's arm and when vibrated signal the user to change his body posture. Similarly, the audio device feedback device can be placed within audible distance of the user (not necessarily attached to the user). An advantageous combination would be e.g. a contactless angle measuring device with an audio feedback signal. In particular this combination does not require any physical contact between the user and the feedback apparatus, which leaves the user unhindered in his working activities.

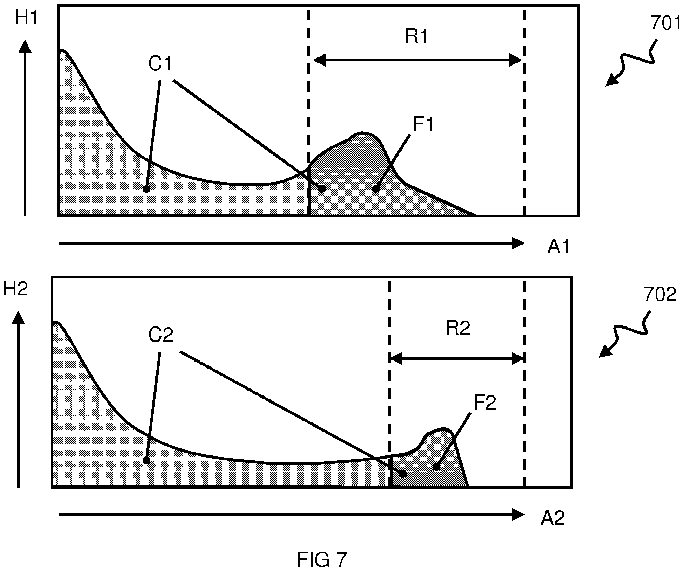

FIG 7 shows histograms 701 and 702 with distributions H1/A1, H2/A2 of relative occurrences H of an angle A between a test subject's arm and body in two subsets of the data C1 and C2. Such a distribution can be obtained e.g. by creating a histogram with bins for different ranges of angles. The distribution represents the relative occurrence of the different angles in the static and dynamic activities. The distribution can be weighted e.g. to the total number of samples that were taken to get an estimate of the fraction of time the test subject was holding his arms at any particular angle. Alternatively, the distribution can also be weighted to the number of samples in the subset. In that case the relative occurrence is indicative of the fraction of time the subject was holding his arms at a particular angle during that type of activity.

To quantify the relative occurrence of angles, evaluation ranges R1 and R2 can be specified and the fractions F1 and F2 which fall within that range can be calculated. In this way it is possible for example to evaluate whether a person is statically holding his hands more than a first threshold fraction (e.g. more than 10 percent of the time) at an angle inside the first evaluation range R1 (e.g. higher than 60 degrees). At the same time it can be evaluated e.g. whether that person was moving his hands dynamically at angles comprised in the second evaluation range R2 (e.g. higher than 90 degrees) for more than a second threshold fraction (e.g. more than 15 percent of the time).

It is noted that it may not be necessary to first create a distribution and then calculate a fraction based on an evaluation range. The fraction could also be calculated directly from the data subsets, e.g. simply by counting the number of sample point that fall within an evaluation range and dividing this number by the total number of sample points. The illustration of a distribution is thus only used to clarify the procedure and should not be construed as limiting the current scope.

A method and system are thus provided for automatic evaluation of a subject's body posture. In particular an angle is recorded as a function of time between a subject's body extremity and a connecting body part or gravitational vector. This time-dependent angle is used to assign data pertaining to different periods of the subject's activity into static and dynamic subsets. For the different subsets or types of activity, different criteria are used to evaluate the subject's body posture, in particular for static periods it is evaluated if a certain angle is exceeded for more than a particular fraction of the time in a time window and for dynamic periods it is evaluated if a number of movements in a certain time window is exceeded. A feedback signal is generated if either evaluation exceeds a respective threshold. Advantageously a method and system are thus provided to both assign and evaluate a subject's activity on the basis of a single time-dependent angle variable and provide real-time feedback.

The various elements of the embodiments as discussed and shown offer certain advantages, such as providing a feedback for both static and dynamic body postures. Of course, it is to be appreciated that any one of the above embodiments or processes may be combined with one or more other embodiments or processes to provide even further improvements in finding and matching designs and advantages. It is appreciated that this invention offers particular advantages for systems for evaluation of static or dynamic arm movements, and in general can be applied for any type of movement involving the measurements of angles and assignment into static and dynamic periods.

Further advantages may be achieved by measuring an angle of a subject's elbow, e.g. between a lower arm and a reference direction e.g. along a length of an upper arm or a gravitational vector. Still further advantages may be achieved by recording an angle of a wrist, e.g. measured between a length of a hand and a reference direction along a lower arm or between a length of a hand and reference direction along a gravitational vector. In general the current invention may find application when measuring or recording one or more angles that a body extremity such as a head, arm, lower arm, hand, leg, lower leg, and/or foot, makes with respect to a reference direction which may be measured along a connecting body part, connected to said extremity by a body joint such as a shoulder, elbow, wrist, knee, etcetera. Alternatively the reference direction may be a gravitational vector and the angle may be measured e.g. by a tilt sensor.

Finally, the above-discussion is intended to be merely illustrative of the present system and should not be construed as limiting the appended claims to any particular embodiment or group of embodiments. Thus, while the present system has been described in particular detail with reference to specific exemplary embodiments thereof, it should also be appreciated that numerous modifications and alternative embodiments may be devised by those having ordinary skill in the art. The specification and drawings are accordingly to be regarded in an illustrative manner and are not intended to limit the scope of the appended claims.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise; no specific sequence of acts or steps is intended to be required unless specifically indicated; and no specific ordering of elements is intended to be required unless specifically indicated.

## Claims

1. Apparatus for generating feedback on a test subject's body posture, the apparatus comprising:
- a memory (603) arranged for storage and retrieval of motion data (D) and a plurality of parameters (PAR) used in processing and evaluation of the motion data (D);
- a clock (602) arranged for providing an indication of time (T);
- a motion recording device (601) arranged for recording the motion data (D) into the memory (603), the motion data (D) including an angle between a direction (Ve) substantially along a length of a test subject's body extremity (201a, 201b) and a reference direction (Vt) as a function of the time (T) provided by the clock (602);
- a processor (604) arranged for retrieving the motion data (D) and parameters (PAR) from the memory (603); and
- a feedback device (605) arranged for generating one or more of a visual, audio, or haptic feedback signal (Sf) and controlled by the processor (604), **characterized in that** said processor (604) is programmed to execute the steps of
∘ retrieving the data (D) and parameters (PAR) from the memory (603);
∘ assigning the motion data (D) into a first or second subset (C1, C2), the first subset (C1) defined as motion data (D) wherein the angle (A) remains within a threshold angle bandwidth (B1) for a period longer than a first threshold time period (T1); the second subset (C2) defined as motion data not belonging to the first subset;
∘ calculating (104a), for data assigned to the first subset in a first time window (W1), a fraction of time (F1) wherein the angle (A) exceeds a first threshold angle (R1) and/or calculating (104b), for data assigned to the second subset in a second time window (W2), a number of occurrences (N2) wherein the angle (A) crosses a second threshold angle (R2); and
∘ controlling the feedback device (605) to generate the feedback signal (Sf) if either the fraction of time (F1) exceeds a threshold time fraction (Fm) or the number of occurrences (N2) exceeds an occurrence threshold (Nm).

2. Apparatus according to claim 1, wherein the processor is arranged to control the feedback device to also generate a feedback signal (Sf) if the angle remains within the threshold angle bandwidth (B1) for a period longer than a second threshold time period (T2);

3. Apparatus according to any of the previous claims, wherein the processor is arranged to control the feedback device to also generate a feedback signal (Sf) if a frequency of a repeated increase and/or decrease in angle in the second subset exceeds a frequency threshold.

4. Apparatus according to any of the previous claims, further comprising a filtering device, arranged for filtering the data prior to the calculating step (104a, 104b) to store or keep only data points in the memory that immediately precede (301) or follow (302) a change in angle (A) that is more than the threshold angle bandwidth (B1) within the first threshold time periods (T1).

5. Apparatus according to any of the previous claims, further comprising a display, controlled by the processor for displaying a feedback graph, the graph representing a simplified movement pattern of the test subject that is generated by the processor from the data by displaying data points on the display that are used for assigning the data in the first or second subsets (C1, C2).

6. Apparatus according to any of the previous claims, wherein the motion recording device (601) comprises
- a remote sensor arranged for recording data on the test subject's body posture without contacting the test subject and
- conversion logic arranged for extracting from the recorded data on the test subject's body posture, data comprising the time-dependent angle between the test subject's body extremity and the subject's trunk or gravitational vector.

7. Apparatus according to claim 6 wherein the remote sensor comprises at least two imaging devices spatially separated to capture two or more perspective images of the test subject.

8. Apparatus according to any of the previous claims wherein the motion recording device (601) comprises a motion capture device arranged for recording the test subject's three-dimensional body posture and extracting from the recorded body posture the angle between the test subject's body extremity and the subject's trunk or gravitational vector.

9. Apparatus according to any of the previous claims, wherein the feedback device comprises a display device providing the user with a visual feedback signal.

10. Apparatus according to any of the previous claims, wherein the feedback device comprises a vibration device and/or a sound-generator arranged for providing the user with a haptic and/or audio feedback signal, respectively.

11. Apparatus according to any of the previous claims wherein the body extremity is at least one of a head, arm, lower arm, hand, finger, leg, lower leg, or foot.

12. Apparatus according to any of the previous claims, wherein the reference direction (Vt) is one of a gravitational vector or a vector measured substantially along a length of a trunk (202) or other body part connected to the body extremity by a body joint.

13. Apparatus according to any of the previous claims, wherein the test subject is a virtual person, the apparatus further comprising a simulation module coupled to the motion recording device, the simulation module arranged to generate the motion data (D).

14. Method for generating feedback on a test subject's body posture (P), the method comprising the steps of:
- recording motion data (D) including a time-dependent (T) angle (A) measured between a direction (Ve) substantially along a length of a test subject's body extremity and a reference direction; the method **characterized by**
- assigning the data into a first or second subset (C1, C2), the first subset defined as data wherein the angle (A) remains within a threshold angle bandwidth (B1) for a period longer than a first threshold time period (T1); the second subset defined as data not belonging to the first subset;
- calculating (104a), for data assigned to the first subset of the data in a first time window (W1), a time fraction (F1) wherein the angle (A) exceeds a first threshold angle (R1) and/or calculating (104b), for data belonging to the second subset of the data in a second time window (W2), a number of occurrences (N2) wherein the angle (A) crosses a second threshold angle (R2); and
- generating one or more of a visual, audio, or haptic feedback signal (Sf) if the time fraction (F1) exceeds a threshold time fraction (Fm) or the number of occurrences (N2) exceeds an occurrence threshold (Nm).

15. Method according to claim 14, wherein the first and second threshold angles (R1, R2) are determined from a correlation between a control subject's statistical chance for body injury and a distribution of recorded angles of the control subject performing a control task.

## Patentansprüche

1. Vorrichtung zur Erzeugung von Feedback über die Körperhaltung einer Versuchsperson, die Vorrichtung umfassend:
- einen Speicher (603), angeordnet zur Speicherung und Wiedergewinnung von Bewegungsdaten (D) und einer Vielzahl von Parametern (PAR), verwendet in der Verarbeitung und Beurteilung der Bewegungsdaten (D);
- eine Uhr (602), angeordnet zur Bereitstellung einer Zeitangabe (T);
- eine Bewegungsaufzeichnungsvorrichtung (601), angeordnet zur Aufzeichnung der Bewegungsdaten (D) in dem Speicher (603), die Bewegungsdaten (D) umfassend einen Winkel zwischen einer Richtung (Ve) im Wesentlichen entlang einer Länge einer Körperextremität einer Versuchsperson (201a, 201b) und einer Referenzrichtung (Vt) in Abhängigkeit der von der Uhr (602) bereitgestellten Zeit (T);
- einen Prozessor (604), angeordnet zur Wiedergewinnung der Bewegungsdaten (D) und der Parameter (PAR) aus dem Speicher (603); und
- eine Feedback-Vorrichtung (605), angeordnet zur Erzeugung von einem oder mehreren eines sichtbaren, hörbaren oder tastbaren Feedback-Signals (Sf) und gesteuert von dem Prozessor (604),
**dadurch gekennzeichnet, dass** der Prozessor (604) programmiert ist zur Ausführung der folgenden Schritte:
o Wiedergewinnung der Daten (D) und der Parameter (PAR) aus dem Speicher (603);
o Zuweisung der Bewegungsdaten (D) an einen ersten oder zweiten Teilsatz (C1, C2), der erste Teilsatz (C1) als definiert Bewegungsdaten (D), wobei der Winkel (A) während einer Zeitdauer länger als eine erste Schwellenwertzeitdauer (T1) innerhalb einer Schwellenwertwinkelbandbreite (B1) bleibt; wobei der zweite Teilsatz (C2), definiert als Bewegungsdaten, die nicht zu dem ersten Teilsatz gehören;
∘ Berechnung (104a) eines Zeitbruchteils (F1) für Daten, die dem ersten Teilsatz in einem ersten Zeitfenster (W1) zugewiesen sind, wobei der Winkel (A) einen ersten Schwellenwertwinkel (R1) überschreitet, und/oder Berechnung (104b) einer Anzahl von Vorkommen (N2) für Daten, die dem zweiten Teilsatz in einem zweiten Zeitfenster (W2) zugewiesen sind, wobei der Winkel (A) einen zweiten Schwellenwertwinkel (R2) kreuzt; und
∘ Steuerung der Feedback-Vorrichtung (605), um das Feedback-Signal (Sf) zu erzeugen, wenn der Zeitbruchteil (F1) einen Schwellenwertzeitbruchteil (Fm) überschreitet oder wenn die Anzahl von Vorkommen (N2) einen Vorkommenschwellenwert (Nm) überschreitet.

2. Vorrichtung nach Anspruch 1, wobei der Prozessor angeordnet ist, die Feedback-Vorrichtung zu steuern, um auch ein Feedback-Signal (Sf) zu erzeugen, wenn der Winkel während einer Dauer länger als eine zweite Schwellenwertzeitdauer (T2) innerhalb der Schwellenwertwinkelbandbreite (B1) bleibt.

3. Vorrichtung nach einem der vorhergehenden Ansprüche, wobei der Prozessor angeordnet ist, die Feedback-Vorrichtung zu steuern, um auch ein Feedback-Signal (Sf) zu erzeugen, wenn eine Frequenz einer wiederholten Zu- und/oder Abnahme im Winkel in dem zweiten Teilsatz einen Frequenzschwellenwert überschreitet.

4. Vorrichtung nach einem der vorhergehenden Ansprüche, ferner umfassend eine Filtervorrichtung, angeordnet zum Filtern der Daten vor dem Berechnungsschritt (104a, 104b), um nur Datenpunkte in dem Speicher zu speichern oder zu behalten, die einer Änderung des Winkels (A), die mehr als die Schwellenwertwinkelbandbreite (B1) innerhalb der ersten Schwellenwertzeitdauern (T1) beträgt, unmittelbar vorangehen (301) oder unmittelbar auf diese folgen (302).

5. Vorrichtung nach einem der vorhergehenden Ansprüche, ferner umfassend eine von dem Prozessor gesteuerte Anzeige zum Anzeigen einer Feedback-Grafik, die ein vereinfachtes Bewegungsmuster der Versuchsperson darstellt, das von dem Prozessor anhand der Daten erzeugt wird, indem Datenpunkte, die zur Zuweisung der Daten in den ersten oder zweiten Teilsätzen (C1, C2) verwendet werden, auf der Anzeige angezeigt werden.

6. Vorrichtung nach einem der vorhergehenden Ansprüche, wobei die Bewegungsaufzeichnungsvorrichtung (601) Folgendes umfasst:
- einen Fernsensor, angeordnet zum Aufzeichnen von Daten über die Körperhaltung einer Versuchsperson ohne die Versuchsperson zu berühren, und
- eine Konvertierungslogik, angeordnet zum Entnehmen der aufgezeichneten Daten über die Körperhaltung der Versuchsperson, die Daten umfassend den zeitabhängigen Winkel zwischen der Körperextremität der Versuchsperson und dem Rumpf- oder Gravitationsvektor der Person.

7. Vorrichtung nach Anspruch 6, wobei der Fernsensor mindestens zwei räumlich getrennte Bildgebungsvorrichtungen umfasst, um zwei oder mehr Perspektivbilder der Versuchsperson zu erfassen.

8. Vorrichtung nach einem der vorhergehenden Ansprüche, wobei die Bewegungsaufzeichnungsvorrichtung (601) eine Bewegungserfassungsvorrichtung umfasst, angeordnet zum Aufzeichnen der dreidimensionalen Körperhaltung der Versuchsperson und Entnehmen des Winkels zwischen der Körperextremität der Versuchsperson und des Rumpf- oder Gravitationswinkels der Person anhand der aufgezeichneten Körperhaltung.

9. Vorrichtung nach einem der vorhergehenden Ansprüche, wobei die Feedback-Vorrichtung eine Anzeigevorrichtung umfasst, die dem Benutzer ein sichtbares Feedback-Signal bereitstellt.

10. Vorrichtung nach einem der vorhergehenden Ansprüche, wobei die Feedback-Vorrichtung eine Vibrationsvorrichtung und/oder einen Schallgeber umfasst, angeordnet um dem Benutzer ein tastbares und/oder hörbares Feedback-Signal bereitzustellen.

11. Vorrichtung nach einem der vorhergehenden Ansprüche, wobei die Körperextremität mindestens eines von Kopf, Arm, Unterarm, Hand, Finger, Bein, Unterschenkel oder Fuß ist.

12. Vorrichtung nach einem der vorhergehenden Ansprüche, wobei die Referenzrichtung (Vt) eine ist von einem Gravitationsvektor oder einem Vektor, gemessen im Wesentlichen entlang einer Länge eines Rumpfes (202) oder anderen Körperteils, verbunden mit der Körperextremität durch ein Körpergelenk.

13. Vorrichtung nach einem der vorhergehenden Ansprüche, wobei die Versuchsperson eine virtuelle Person ist, die Vorrichtung ferner umfassend ein Simulationsmodul, gekoppelt an die Bewegungsaufzeichnungsvorrichtung, das Simulationsmodul angeordnet zum Erzeugen der Bewegungsdaten (D).

14. Verfahren zur Erzeugung von Feedback über die Körperhaltung (P) einer Versuchsperson, das Verfahren umfassend folgende Schritte:
- Aufzeichnung von Bewegungsdaten (D), einschließlich eines zeitabhängigen (T) Winkels (A), gemessen zwischen einer Richtung (Ve) im Wesentlichen entlang einer Länge einer Körperextremität einer Versuchsperson und einer Referenzrichtung; das Verfahren **gekennzeichnet durch**
- Zuweisung der Daten zu einem ersten oder zweiten Teilsatz (C1, C2), der erste Teilsatz definiert als Daten, wobei der Winkel (A) während einer Zeitdauer länger als eine erste Schwellenwertzeitdauer (T1) innerhalb einer Schwellenwertwinkelbandbreite (B1) bleibt; der zweite Teilsatz definiert als Daten, die nicht zu dem ersten Teilsatz gehören;
- Berechnung (104a) eines Zeitbruchteils (F1) für Daten, die dem ersten Teilsatz der Daten in einem ersten Zeitfenster (W1) zugewiesen sind, wobei der Winkel (A) einen ersten Schwellenwertwinkel (R1) überschreitet, und/oder Berechnung (401b) einer Anzahl von Vorkommen (N2) für Daten, die zu dem zweiten Teilsatz der Daten in einem zweiten Zeitfenster (W2) gehören, wobei der Winkel (A) einen zweiten Schwellenwertwinkel (R2) kreuzt; und
- Erzeugung von einem oder mehreren eines sichtbaren, hörbaren oder tastbaren Feedback-Signals (Sf), wenn der Zeitbruchteil (F1) einen Schwellenwertzeitbruchteil (Fm) überschreitet oder die Anzahl der Vorkommen (N2) einen Vorkommenschwellenwert (Nm) überschreitet.

15. Verfahren nach Anspruch 14, wobei die ersten und zweiten Schwellenwertwinkel (R1, R2) anhand einer Korrelation zwischen dem statistischen Risiko auf Körperverletzung einer Kontrollperson und einer Verteilung aufgezeichneter Winkel der Kontrollperson, die eine Kontrollaufgabe ausführt, bestimmt werden.

## Revendications

1. Appareil destiné à produire une rétroaction concernant une posture corporelle d'un sujet de test, l'appareil comprenant :
- une mémoire (603) agencée pour stocker et récupérer des données de mouvement (D) et plusieurs paramètres (PAR) utilisés dans un traitement et une évaluation des données de mouvement (D) ;
- une horloge (602) agencée pour fournir une indication de temps (T) ;
- un dispositif d'enregistrement de mouvement (601) agencé pour enregistrer les données de mouvement (D) dans la mémoire (603), les données de mouvement (D) comprenant un angle situé entre une direction (Ve) sensiblement dans la longueur d'une extrémité corporelle du sujet de test (201a, 201b) et une direction de référence (Vt) en fonction du temps (T) fournie par l'horloge (602) ;
- un processeur (604) agencé pour récupérer les données de mouvement (D) et les paramètres (PAR) dans la mémoire (603) ; et
- un dispositif de rétroaction (605) agencé pour produire un ou plusieurs signaux parmi un signal de rétroaction visuel, audio, ou haptique (Sf) et commandé par le processeur (604), **caractérisé en ce que** ledit processeur (604) est programmé pour exécuter les étapes consistant à :
∘ récupérer les données (D) et les paramètres (PAR) dans la mémoire (603) ;
∘ transférer les données de mouvement (D) dans un premier et/ou second sous-ensemble (C1, C2), le premier sous-ensemble (C1) étant défini comme des données de mouvement (D) dans lesquelles l'angle (A) reste dans une largeur de bande d'angle de seuil (B1) pendant une période plus longue qu'une première période de temps de seuil (T1) ; le second sous-ensemble (C2) étant défini comme des données de mouvement n'appartenant pas au premier sous-ensemble ;
∘ calculer (104a), pour des données transférées au premier sous-ensemble dans une première fenêtre de temps (W1), une fraction de temps (F1) dans laquelle l'angle (A) dépasse un premier angle de seuil (R1) et/ou calculer (104b), pour des données transférées au second sous-ensemble dans une seconde fenêtre de temps (W2), un nombre d'occurrences (N2) dans lesquelles l'angle (A) recoupe un second angle de seuil (R2) ; et
∘ commander le dispositif de rétroaction (605) pour produire le signal de rétroaction (Sf) si la fraction de temps (F1) dépasse une fraction de temps de seuil (Fm) ou si le nombre d'occurrences (N2) dépasse un seuil d'occurrence (Nm).

2. Appareil selon la revendication 1, dans lequel le processeur est agencé pour commander le dispositif de rétroaction pour produire également un signal de rétroaction (Sf) si l'angle reste dans la largeur de bande d'angle de seuil (B1) pendant une période plus longue qu'une seconde période de temps de seuil (T2) ;

3. Appareil selon l'une quelconque des revendications précédentes, dans lequel le processeur est agencé pour commander le dispositif de rétroaction pour fournir également un signal de rétroaction (Sf) si une fréquence d'augmentation et/ou diminution répétée d'un angle dans le second sous-ensemble dépasse un seuil de fréquence.

4. Appareil selon l'une quelconque des revendications précédentes, comprenant de plus un dispositif de filtrage, agencé pour filtrer les données avant l'étape de calcul (104a, 104b) pour mémoriser ou garder uniquement des points de données dans la mémoire qui précèdent immédiatement (301) ou suivent immédiatement (302) un changement de l'angle (A) qui est plus grand que la largeur de bande d'angle de seuil (B1) dans les premières périodes de temps de seuil (T1).

5. Appareil selon l'une quelconque des revendications précédentes, comprenant de plus un affichage, commandé par le processeur pour afficher un graphique de rétroaction, le graphique représentant un motif de mouvement simplifié du sujet de test qui est produit par le processeur à partir des données en affichant des points de données sur l'affichage, qui sont utilisés pour transférer les données dans les premier ou second sous-ensemble (C1, C2).

6. Appareil selon l'une quelconque des revendications précédentes, dans lequel le dispositif d'enregistrement de mouvement (601) comprend
- un capteur distant agencé pour enregistrer des données concernant la posture du corps du sujet de test sans contact avec le sujet de test, et
- une logique de conversion agencée pour extraire à partir des données enregistrées concernant la posture du corps du sujet de test, des données comprenant l'angle en fonction du temps entre l'extrémité du corps du sujet de test et le tronc du sujet, ou un vecteur gravitationnel.

7. Appareil selon la revendication 6, dans lequel le capteur distant comprend au moins deux dispositifs d'imagerie séparés spatialement pour capturer deux ou plus de deux images en perspective du sujet de test.

8. Appareil selon l'une quelconque des revendications précédentes, dans lequel le dispositif d'enregistrement de mouvement (601) comprend un dispositif de capture de mouvement agencé pour enregistrer la posture corporelle tridimensionnelle du sujet et extraire à partir de la posture corporelle enregistrée l'angle entre l'extrémité du corps du sujet de test et le tronc du sujet, ou un vecteur gravitationnel.

9. Appareil selon l'une quelconque des revendications précédentes, dans lequel le dispositif de rétroaction comprend un dispositif d'affichage fournissant à l'utilisateur un signal de rétroaction visuel.

10. Appareil selon l'une quelconque des revendications précédentes, dans lequel le dispositif de rétroaction comprend un dispositif de vibration et/ou un générateur sonore agencé pour fournir à l'utilisateur un signal de rétroaction haptique et/ou audio, respectivement.

11. Appareil selon l'une quelconque des revendications précédentes, dans lequel l'extrémité du corps est au moins une partie parmi la tête, un bras, un bras inférieur, une main, un doigt, une jambe, une jambe inférieure ou un pied.

12. Appareil selon l'une quelconque des revendications précédentes, dans lequel la direction de référence (Vt) est un vecteur parmi un vecteur gravitationnel ou un vecteur mesuré sensiblement dans la longueur du tronc (202) ou d'une autre partie corporelle reliée à l'extrémité de corps par une articulation corporelle.

13. Appareil selon l'une quelconque des revendications précédentes, dans lequel le sujet de test est une personne virtuelle, l'appareil comprenant de plus un module de simulation relié au dispositif d'enregistrement de mouvement, le module de simulation étant agencé pour produire les données de mouvement (D).

14. Méthode pour produire une rétroaction concernant une posture corporelle de sujet de test (P), la méthode comprenant les étapes consistant à :
- enregistrer des données de mouvement (D) incluant un angle (A) en fonction du temps (T) mesuré entre une direction (Ve) sensiblement le long d'une longueur d'une extrémité corporelle du corps du sujet de test et une direction de référence, la méthode étant **caractérisée par** les étapes consistant à :
- transférer les données dans un premier ou second sous-ensemble (C1, C2), le premier sous-ensemble étant défini comme des données dans lesquelles l'angle (A) reste dans une largeur de bande d'angle de seuil (B1) pendant une période plus longue qu'une première période de temps de seuil (T1) ; le second sous-ensemble étant défini comme des données n'appartenant pas au premier sous-ensemble ;
- calculer (104a), pour des données transférées au premier sous-ensemble dans une première fenêtre de temps (W1), une fraction de temps (F1) dans laquelle l'angle (A) dépasse un premier angle de seuil (R1) et/ou calculer (104b), pour des données appartenant au second sous-ensemble des données dans une seconde fenêtre de temps (W2), un nombre d'occurrences (N2) dans lesquelles l'angle (A) recoupe un second angle de seuil (R2) ; et
- produire un ou plusieurs signaux parmi un signal de rétroaction visuel, audio, ou haptique (Sf) si la fraction de temps (F1) dépasse une fraction de temps de seuil (Fm) ou si le nombre d'occurrences (N2) dépasse un seuil d'occurrence (Nm).

15. Méthode selon la revendication 14, dans laquelle les premier et second angles de seuil (R1, R2) sont déterminés à partir d'une corrélation entre un risque statistique pour le sujet témoin de blessure corporelle et une répartition des angles enregistrés du sujet témoin effectuant une tâche de témoin.
